# EUROPÄISCHE PATENTSCHRIFT

(11) **EP 1 498 088 B1**
(45) Veröffentlichungstag und Bekanntmachung des Hinweises auf die Patenterteilung: **22.10.2014**
(21) Anmeldenummer: 04400036.2
(22) Anmeldetag: 06.07.2004
(51) Int. Cl.: A61F 2/18, A61F 11/04

(54) **Gehörknöchelchenprothese**
Flexible ossicular prosthesis
Prothèse d'osselets flexible

(30) Priorität: 08.07.2003 DE 10331644
(43) Veröffentlichungstag der Anmeldung: 19.01.2005
(73) Patentinhaber: Heinz Kurz GmbH Medizintechnik, 72144 Dusslingen (DE)
(72) Erfinder: Hüttenbrink, Karl-Bernd, 01326 Dresden (DE); Zahnert, Thomas, 01326 Dresden (DE); Hofmann, Gert, 01465 Dresden-Langebrück (DE); Bornitz, Matthias, 01157 Dresden (DE)
(74) Vertreter: Möbus, Daniela

(56) Entgegenhaltungen:
- DE-A1- 19 647 579
- DE-A1- 19 923 403
- DE-A1- 19 935 029
- DE-A1- 19 948 336
- DE-A1- 19 948 375
- DE-U1- 20 014 659
- US-A- 3 196 462
- US-A- 4 624 672
- US-B1- 6 203 571

## Beschreibung

Die Erfindung betrifft eine Gehörknöchelchenprothese zur Übertragung von Schallschwingungen im Mittelohr unter Berücksichtigung des sich ändernden statischen Drucks oder größerer momentaner Druckbelastungen, wie z. B. beim Niesen. Die erfindungsgemäße Gehörknöchelchenprothese umfasst Anschlusselemente zum Trommelfell und zum ovalen Fenster des Innenohrs bzw. zu den an diese angeschlossenen, noch vorhandenen Reste der Gehörknöchelchen, wie insbesondere Hammergriff und Steigbügel oder deren zur Operation noch verwendbare Teile.

Eine derartige Gehörknöchelchenprothese ist in der US 6,203,571 B1 beschrieben.

Es sind bereits Gehörknöchelchenprothesen bekannt, die nicht nur ihre eigentliche Funktion, die Übertragung von Schallschwingungen, erfüllen, sondern auch in der Lage sind Abstandsänderungen zwischen Trommelfell und ovalem Fenster des Innenohrs durch statische oder momentan größere Druckschwankungen zu folgen.

Nach US 4,957,507 A besteht die Prothese im wesentlich aus einem Federdrahtwinkel. Einer seiner Arme liegt etwa parallel zum Trommelfell und nimmt die Abstandsänderungen auf. Der andere ist etwa senkrecht auf das ovale Fenster gerichtet und überträgt die Schallschwingungen. Es ist schwierig, die Federsteifigkeiten beider Federdrahtarme unabhängig voneinander auf ihre jeweiligen spezifischen Aufgaben hin zu optimieren.

Auch die Lösung nach DE 196 47 579 A1 zeichnet sich dadurch aus, dass das Mittelohrimplantat über mindestens zwei Abschnitte mit unterschiedlichen Elastizitätseigenschaften verfügt, die senkrecht aufeinander stehen. Prinzipiell können beide Bereiche weitgehend unabhängig voneinander optimiert werden. Die Herstellung ist aber insgesamt aufwendig. Es bleibt wenig Raum für notwendige Anpassungen an die gegebenen Abmessungen und sonstigen Bedingungen im Mittelohr des jeweiligen Patienten. DE 196 47 579 A1 zeigt eine einstückig ausgeführte Gehörknöchelchenprothese mit einem annähernd geraden und einem abgewinkelten hebelartigen Verbindungsteil. Als Federelement ist ein relativ kompliziertes, labyrinthartig aufgebautes federndes Zwischenteil vorgesehen, dessen Außengehäuse selbst in der Paukenhöhle fixiert wird.

Die in der US 4,624,672 A vorgeschlagene Mittelohrprothese ist ebenfalls einstückig aus einem einzigen, an verschiedenen Stellen und mehrfach spulenartig gewickelten Edelstahldraht aufgebaut. Bei dieser Prothese mag vielleicht eine - wenn überhaupt, dann bestenfalls nur sehr begrenzte - Gelenkigkeit und Elastizität über die spezielle Ausformung des die einstückige Prothese bildenden Drahtes erzielt werden. Letzterer ist - vermutlich zu diesem Zweck - in einem mittleren Abschnitt schraubenförmig gewendelt und läuft dann beiderseits der dadurch gebildeten Schraubenfeder jeweils stabartig zu den beiden Anschlusselementen weiter, die ebenfalls einstückig aus diesem Draht geformt sind.

In US 3,710,399 A und DE 200 14659 U1 bestehen die Prothesen jeweils aus zwei stabförmigen Teilen, die über eine Art Schiebemuffe bzw. mit einem Kugelgelenk miteinander verbunden sind. Ihre Länge bzw. die gegenseitige Winkellage der Anschlüsse kann gut auf Gegebenheiten im Ohr des jeweiligen Patienten eingestellt werden.

In DE 199 35 029 A1 ist zwischen zwei stabförmige Prothesenteile ein Dämpfungsglied mit entropieelastischen Eigenschaften geschaltet. Mit ihm wird die Charakteristik der Schallübertragung modifiziert. DE 199 35 029 A1 beschreibt eine Anordnung zum Ankoppeln eines elektromechanischen Schallwandlers an ein Gehörknöchelchen, bei der ein stabartiges Schallübertragungselement unterbrochen und an der Unterbrechungsstelle über ein Dämpfungsglied mit "Entropie-elastischen Eigenschaften" verbunden ist. Zwar sind hier formal getrennte "Stabteile" vorhanden, aber "federnde" Eigenschaften des dazwischen liegenden "Dämpfungsgliedes" sind nicht offenbart.

Ähnliches gilt auch für die in der DE 199 23 403 A1 beschriebene "Vorrichtung zum mechanischen Ankoppeln eines in einer Mastoidhöhle implantierbaren elektromechanischen Hörgerätewandlers".Die eingangs zitierte US 6,203,571 B1 offenbart eine Mittelohrprothese, die aus einem einstückigen, blattfederartigen Teil besteht, bei dem lediglich die beiden Anschlussstellen, die im menschlichen Mittelohr eine Verbindung zum Hammer einerseits und zum Steigbügel andererseits herstellen sollen, entsprechend gebogen bzw. abgewinkelt sind.

DE19948375 offenbart zwei stabförmige Prothesenteile, entsprechend des Oberbegriffs von Anspruch 1, die mittels eines Koppelelements zu einer Gehörknöchelchenprothese verbunden sind.

Aufgabe der Erfindung ist es demgegenüber, eine weitere Gehörknöchelchenprothese zur Übertragung von Schallschwingungen im Mittelohr unter Berücksichtigung des sich ändernden statischen Drucks oder größerer momentaner Druckbelastungen anzugeben, die einfach herstell- und anpassbar ist.

Erfindungsgemäß wird diese Aufgabe dadurch gelöst, dass zwei miteinander verbundene stabartige Glieder zur Übertragung des Schalls zwischen Trommelfell und ovalem Fenster vorgesehen und beide Stabteile über ein Federgelenk miteinander verbunden sind, dass das Federgelenk eine elastische Kugel oder eine elastische Hülse ist, dass wenigstens eine der im Federgelenk liegenden Stirnseiten der Stabteile ballig ist und die Stirnseiten aufeinander abrollen, und dass das Federgelenk aus einem Silikonmaterial gefertigt ist.

Der Schall wird von den beiden Stabteilen und dem sie verbindende Federgelenk longitudinal übertragen. Bei Abstandsänderungen knicken die beiden Stabteile im Federgelenk seitlich aus, wobei der Schall weiterhin in nahezu unveränderter Qualität weitergeleitet wird.

Die Prothese kann aus biologisch verträglichen Materialien, wie insbesondere Titan für alle festen Teile und Silikon für das Gelenk, hergestellt werden. In einfachster Weise können derzeit bereits in Fertigung befindliche Gehörknöchelchenprothesen mit einem stabartigen Übertragungsglied geändert werden. Die endgültige Länge der Stabteile kann noch vor Ort angepasst werden.

Im entspannten Zustand schließen die Stabteile einen stumpfen Winkel ein. Die Prothese lässt sich in diesem Zustand leichter ausknicken. Die Richtung des Ausknickens ist in Anpassung an die Anatomie des Ohres von vornherein vorgegeben. Außerdem besteht dadurch auch eine kleine Reserve, um Abstandsvergrößerungen zwischen Trommelfell und ovalem Fenster zu folgen.

Die weiteren Unteransprüche und die nachfolgenden Beispiele enthalten spezielle Ausgestaltungen und Erläuterungen zu den Merkmalen der Erfindung.

In den Zeichnungen zeigen
Fig. 1 und 2 eine erste erfindungsgemäße Ausführung und
Figuren 3 bis 5 jeweils einen Ausschnitt durch verschiedene erfindungsgemäße Federgelenkgestaltungen.

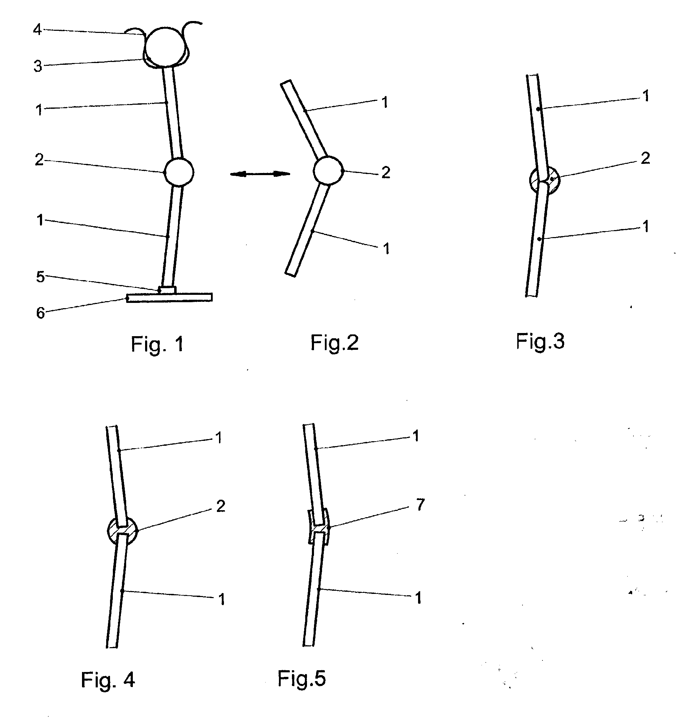

In Fig. 1 ist eine Gehörknöchelchenprothese dargestellt, die mit einem Federkorb 4 am Hammergriff 3 angeschlossen ist und über ein Füßchen 5 und eine Platte 6 auf dem (nicht dargestellten) Steigbügel aufsetzt.

Zwischen dem Federkorb 4 und dem Füßchen 5 befinden sich zwei den Schall übertragende Stabteile 1, die über ein Federgelenk 2 miteinander verbunden sind.

Im frisch eingesetzten Zustand bzw. bei normalem Atmosphärendruck schließen die Stabteile 1 einen stumpfen Winkel ein. Sie sind bereits etwas ausgeknickt. In Fig. 2 wird gezeigt, wie die Stabteile unter höherem Druck seitlich weiter ausknicken. Die Prothese kann damit momentanen oder länger anhaltenden Abstandsänderungen zwischen dem Hammergriff 3 und dem Steigbügel nahezu problemlos folgen, ohne dass die Schallübertragungen unterbrochen oder wesentlich beeinträchtigt wird. Um diese optimale Schallübertragung zu gewährleisten, dürfen die elastischen Rückstellkräfte der ausgeknickten Prothese 10 mN nicht überschreiten. Die Richtung des Ausknickens ist durch den stumpfen Winkel vorgegeben. Sie ist der Anatomie des Mittelohres angepasst.

In Fig. 1 ist das Federgelenk mittels einer Silikonkugel 2 realisiert. Die Figuren 3 und 4 zeigen zwei verschiedene Ausführungsformen dieser Variante. In Fig. 3 stoßen die Stirnseiten der Stabteile 1 aufeinander. Sie sind ballig ausgeführt, um den Winkeländerungen zu folgen. In Fig. 4 befindet sich ein Teil des Silikons zwischen den Stirnseiten der Stabteile 1. Der Schall wird auch hierbei noch sehr gut übertragen.

In Fig. 5 ist das Federgelenk eine elastische Hülse 7. Zwischen den Stirnseiten der Stabteile 1 befindet sich wie bei der Ausführung nach Fig. 4 Silikon.

Die Stablängen können gegebenenfalls noch vor Ort den Gegebenheiten des Mittelohrs, in das die Prothese eingesetzt werden, soll angepasst werden. Das bezieht sich nicht nur auf die Länge der Stabteile 1, sondern auch auf die Anschlusselemente Federkorb 4 bzw. Füßchen 5 und Platte 6. Beispielsweise kann in Fig. 1 der obere Stab 1 mit Federkorb 4 unkompliziert gegen einen Stab 1 mit einem bekannten Anschlusselement, das direkt am Trommelfell anliegen soll, ausgetauscht werden.

## Patentansprüche

1. Gehörknöchelchenprothese zur Übertragung von Schallschwingungen im Mittelohr mit Anschlusselementen zum Trommelfell und zum ovalen Fenster des Innenohrs bzw. zu den an diese angeschlossenen, noch vorhandenen Resten der Gehörknöchelchen, wie insbesondere Hammergriff und Steigbügel oder deren zur Operation noch verwendbare Teile, mittels zwei miteinander verbundenen stabartigen Glieden zur Übertragung des Schalls zwischen Trommelfell und ovalem Fenster vorgesehen sind, **gekennzeichnet dadurch, dass** beide Stabteile (1) über ein Federgelenk (2 bzw. 7) miteinander verbunden sind, dass das Federgelenk eine elastische Kugel (2) oder eine elastische Hülse (7) ist, dass wenigstens eine der im Federgelenk (2 bzw. 7) liegenden Stirnseiten der Stabteile (1) ballig ist und die Stirnseiten aufeinander abrollen, und dass das Federgelenk (2 bzw. 7) aus einem Silikonmaterial gefertigt ist.

2. Gehörknöchelchenprothese nach Anspruch 1, **gekennzeichnet dadurch, dass** die Stabteile (1) im entspannten Zustand einen stumpfen Winkel einschließen.

3. Gehörknöchelchenprothese nach Anspruch 1, **gekennzeichnet dadurch, dass** die elastischen Rückstellkräfte im Federgelenk bei ausgeknickter Prothese 10 mN nicht überschreiten.

## Claims

1. Auditory ossicle prosthesis for the transmission of sound vibrations in the middle ear, comprising connecting elements to the eardrum and to the oval window of the inner ear or to the remaining portions of the auditory ossicles connected thereto, such as, in particular, manubrium of malleus and stapes or the parts thereof that can still be used for surgery, by means of two rod-like elements connected together provided for the transmission of sound between the eardrum and the oval window, **characterised in that** the two rod elements (1) are connected together via a spring joint (2 or 7), that the spring joint is a flexible ball (2) or a flexible sleeve (7), that at least one of the end faces of the rod elements (1) situated in the spring joint (2 or 7) is spherical and the end faces roll upon one another and that the spring joint (2 or 7) is made of a silicone material.

2. Auditory ossicle prosthesis according to claim 1, **characterised in that** the rod elements (1) include an obtuse angle in the relaxed state.

3. Auditory ossicle prosthesis according to claim 1, **characterised in that** the elastic restoring forces in the spring joint when the prosthesis is bent outwards do not exceed 10 mN.

## Revendications

1. Prothèse pour les osselets destinée à la transmission de vibrations sonores dans l'oreille moyenne avec des éléments de raccordement au tympan et à la fenêtre ovale de l'oreille interne ou respectivement à des restes des osselets encore présents raccordés à ceux-ci, comme en particulier le manche de marteau et l'étrier ou leurs parties encore utilisables pour l'opération, deux éléments du type barre raccordés à l'un à l'autre étant prévus pour la transmission du son entre le tympan et la fenêtre ovale, **caractérisé en ce que** deux parties de barre (1) sont raccordées l'une à l'autre par le biais d'une articulation à ressort (2 ou respectivement 7), **en ce que** l'articulation à ressort est une bille (2) élastique ou un manchon (7) élastique, **en ce qu'**au moins un des côtés frontaux des parties de barre (1) situés dans l'articulation à ressort (2 ou respectivement 7) est sphérique et les côtés frontaux roulent l'un sur l'autre, et **en ce que** l'articulation à ressort (2 ou respectivement 7) est réalisée dans un matériau en silicone.

2. Prothèse pour les osselets selon la revendication 1, **caractérisée en ce que** les parties de barre (1) forment un angle obtus quand elles sont dans l'état détendu.

3. Prothèse pour les osselets selon la revendication 1, **caractérisée en ce que** les forces de rappel élastiques dans l'articulation à ressort ne dépassent pas 10 mN quand la prothèse est pliée.
